# EUROPEAN PATENT SPECIFICATION

(11) **EP 3 280 344 B1**
(45) Date of publication and mention of the grant of the patent: **29.01.2025**
(21) Application number: 16718475.3
(22) Date of filing: 05.04.2016
(51) Int. Cl.: A61B 34/20, A61B 90/00, A61B 90/50, A61B 17/00, A61B 34/10, G02B 27/01, G06T 7/00, A61B 34/00, G06T 7/73, G06T 19/00

(54) **SYSTEM FOR UTILIZING AUGMENTED REALITY TO IMPROVE SURGERY**
SYSTEM ZUR VERWENDUNG VON ERWEITERTER REALITÄT FÜR VERBESSERTE CHIRURGISCHE EINGRIFFE
SYSTÈME D'UTILISATION DE LA RÉALITÉ AUGMENTÉE POUR AMÉLIORER UNE OPÉRATION CHIRURGICALE

(30) Priority: 07.04.2015 US 201562143923 P
(43) Date of publication of application: 14.02.2018
(73) Proprietor: King Abdullah University Of Science And Technology, Thuwal 23955-6900 (SA)
(72) Inventor: CALI, Corrado, Thuwal 23955-6900 (SA); BESUCHET, Jonathan, Thuwal 23955-6900 (SA)
(74) Representative: Ipsilon
(86) International application number: PCT/IB2016/051924
(87) International publication number: WO 2016/162789

(56) References cited:
- WO-A1-2014/200016
- WO-A1-2014/200016
- WO-A1-2014/200016
- US-A1- 2005 203 380
- US-A1- 2005 203 380
- US-A1- 2005 203 380
- US-A1- 2006 176 242
- US-A1- 2006 176 242
- US-A1- 2006 176 242
- US-A1- 2013 267 838
- US-A1- 2013 267 838
- US-A1- 2013 267 838
- US-A1- 2014 275 760
- US-A1- 2014 275 760
- US-A1- 2014 275 760
- US-A1- 2015 049 081
- US-A1- 2015 049 081
- SKRINJAR O M ET AL: "REAL TIME 3D BRAIN SHIFT COMPENSATION", INFORMATION PROCESSING IN MEDICAL IMAGING. INTERNATIONALCONFERENCE. PROCEED, XX, XX, vol. 1613, 25 June 1999 (1999-06-25), pages 42 - 55, XP008010724
- SUN KAY ET AL: "Near Real-Time Computer Assisted Surgery for Brain Shift Correction Using Biomechanical Models", IEEE JOURNAL OF TRANSLATIONAL ENGINEERING IN HEALTH AND MEDICINE, vol. 2, 30 May 2014 (2014-05-30), pages 1 - 13, XP011552671, DOI: 10.1109/JTEHM.2014.2327628
- MATHIAS MARKERT ET AL: "Tracking of the liver for navigation in open surgery", INTERNATIONAL JOURNAL OF COMPUTER ASSISTED RADIOLOGY AND SURGERY, vol. 5, no. 3, 7 August 2009 (2009-08-07), DE, pages 229 - 235, XP055593706, ISSN: 1861-6410, DOI: 10.1007/s11548-009-0395-x
- OLIVEIRA-SANTOS THIAGO ET AL: "Passive Single Marker Tracking for Organ Motion and Deformation Detection in Open Liver Surgery", 22 June 2011, INTERNATIONAL CONFERENCE ON COMPUTER ANALYSIS OF IMAGES AND PATTERNS. CAIP 2017: COMPUTER ANALYSIS OF IMAGES AND PATTERNS; [LECTURE NOTES IN COMPUTER SCIENCE; LECT.NOTES COMPUTER], SPRINGER, BERLIN, HEIDELBERG, PAGE(S) 156 - 167, ISBN: 978-3-642-17318-9, XP047369683
- SKRINJAR O M ET AL: "REAL TIME 3D BRAIN SHIFT COMPENSATION", INFORMATION PROCESSING IN MEDICAL IMAGING. INTERNATIONALCONFERENCE. PROCEED, XX, XX, vol. 1613, 25 June 1999 (1999-06-25), pages 42 - 55, XP008010724
- SUN KAY ET AL: "Near Real-Time Computer Assisted Surgery for Brain Shift Correction Using Biomechanical Models", IEEE JOURNAL OF TRANSLATIONAL ENGINEERING IN HEALTH AND MEDICINE, vol. 2, 30 May 2014 (2014-05-30), pages 1 - 13, XP011552671, DOI: 10.1109/JTEHM.2014.2327628
- MATHIAS MARKERT ET AL: "Tracking of the liver for navigation in open surgery", INTERNATIONAL JOURNAL OF COMPUTER ASSISTED RADIOLOGY AND SURGERY, vol. 5, no. 3, 7 August 2009 (2009-08-07), DE, pages 229 - 235, XP055593706, ISSN: 1861-6410, DOI: 10.1007/s11548-009-0395-x
- OLIVEIRA-SANTOS THIAGO ET AL: "Passive Single Marker Tracking for Organ Motion and Deformation Detection in Open Liver Surgery", 22 June 2011, INTERNATIONAL CONFERENCE ON COMPUTER ANALYSIS OF IMAGES AND PATTERNS. CAIP 2017: COMPUTER ANALYSIS OF IMAGES AND PATTERNS; [LECTURE NOTES IN COMPUTER SCIENCE; LECT.NOTES COMPUTER], SPRINGER, BERLIN, HEIDELBERG, PAGE(S) 156 - 167, ISBN: 978-3-642-17318-9, XP047369683

## Description

### TECHNOLOGICAL FIELD

Example embodiments of the present invention relate generally to computer-assisted surgery and, more particularly, to a system utilizing augmented reality to enhance surgery.

### BACKGROUND

Surgery is in general an invasive procedure; the more invasive it is, the greater the time that is needed for recovery and the greater the probability of post-surgery complications. Medical technology has always looked for improvements to limit the likelihood of post-surgery complications, and to date minimally-invasive surgeries are performed routinely. In this regard, the improvement of digital medical imaging techniques has provided a major boost to the diffusion of minimal invasive procedures. Today, physicians often rely on digital imaging as a tool to plan surgeries, as well as a visual guide during actual surgery, to access sites that would otherwise be hidden from view.

### BRIEF SUMMARY

The inventors have thus discovered that a need exists for enhancements to digital imaging tools that provide visual guides during surgery. To this end, embodiments described herein illustrate methods, apparatus, and systems for utilizing augmented reality, based on the use of three dimensional (3D) medical imaging to enhance the use of surgical tools such as a neuronavigator (a machine able to track the position of surgical probes inside the brain or other operating areas of a patient, and visualize it on MRI or CT scans of the patient itself). As described herein, instead of using a remote screen displaying single plane (i.e., two dimensional or 2D) scans of a target area of a patient (e.g., a body part, a tumor, an area into which a foreign object has been lodged, or the like), a surgeon employing example embodiments described herein may utilize a head-mounted display to perceive the 3D model of a target area during a surgery. This 3D model can be extracted from a scan, and visualized directly as a superimposition onto the actual target area of the patient undergoing surgery using augmented reality glasses.

The invention is as defined in the appended set of claims. It will be appreciated that the scope of the invention encompasses many potential embodiments, some of which will be further described below.

### BRIEF DESCRIPTION OF THE DRAWINGS

Reference will now be made to the accompanying drawings, which are not necessarily drawn to scale, and wherein:
Figure 1 is a block diagram of an example computing device that may comprise or be utilized by example embodiments of the present invention;
Figures 2A-2D provide a series of illustrations regarding operations used to generate a 3D reconstruction of a target area of a patient, in accordance with some example embodiments of the present invention;
Figures 3A-3C provide a series of illustrations that demonstrate the connection between various elements of the present invention, in accordance with some example embodiments;
Figure 4 illustrates a flow chart including example operations performed by a computing device for utilizing three dimensional augmented reality visualization to assist surgery, in accordance with some example embodiments of the present invention;
Figure 5 illustrates a flow chart including example operations performed by a computing device for generating a three dimensional reconstruction of a target area based on an image stack received from a scan of the target area, in accordance with some example embodiments of the present invention;
Figure 6 illustrates a flow chart including example operations performed by a computing device for superimposing a three dimensional augmented reality projection using augmented reality glasses, in accordance with some example embodiments of the present invention;
Figure 7 illustrates a flow chart including example operations performed by a computing device for ensuring accurate alignment of a three dimensional projection based on relative locations and orientations of objects within a user's field of view, in accordance with some example embodiments of the present invention; and
Figure 8 illustrates a flow chart including example operations performed by a computing device for maintaining alignment of a three dimensional projection based on changes in objects within the field of view, in accordance with some example embodiments of the present invention.

### DETAILED DESCRIPTION

Some embodiments of the present invention will now be described more fully hereinafter with reference to the accompanying drawings, in which some, but not all embodiments of the invention are shown. Indeed, the invention may be embodied in many different forms and should not be construed as limited to the embodiments set forth herein; rather, these embodiments are provided so that this disclosure will satisfy applicable legal requirements. Like numbers refer to like elements throughout.

### Overview

Neuronavigation systems are unique tools used to access lesions or tumors or other target areas located far too deep inside the brain for traditional surgical methods and which commonly result in surgical sites that do not have clear visibility or access. In order to provide sufficiently accurate tracking to reach such small targets, the set-up of assistive machinery in the operating room (e.g., a neuronavigator or the like) requires many hours of work before starting the actual surgery. Also, the precision by which surgical tools associated with the neuronavigator can be tracked is limited by the fact that the system cannot take into account the natural movements and deformations (swelling) occurring in the target area (e.g., the brain) during surgery.

Given this background, the use of the assistive machine itself is not very comfortable because the surgeon needs to focus attention on a screen that illustrates the actual position of the probe, while the surgeon's hands are operating on an operatory field that is oriented differently from this field of view. It is not uncommon, for instance, for the orientation of the images on the screen to be the reverse of the physical orientation in the actual working area, a situation that requires an extra level of concentration and may limit a surgeon's operative capabilities. These issues limit the distribution and use of assistive devices such as neuronavigators.

The inventors have identified a need for simplifying the preparation of the operating room, improving the comfort of the surgeon by enabling the direct visualization of the position and the path of the surgical probes superimposed on the real field of view of the surgeon, and improving the tracking of both the surgical instruments and the target areas upon which surgery is performed, in order to proficiently operate on small targets.

To address these needs and others, example embodiments described herein utilize improved digital imaging tools that provide a three dimensional visual guide during surgery. In particular, a head-mounted display provides augmented reality visualizations that improve a surgeon's understanding of the relative locations of a target area within the context of the surgeon's field of view, and, in some embodiments, also illustrates a relative location of the surgeon's surgical tools.

Historical systems and applications for digitally-enhanced surgical applications have not taken advantage of augmented reality and 3D superimposition, and no historical mechanism for enhancing surgery has considered the use of augmented reality 3D glasses as described herein. Using augmented reality 3D glasses, example embodiments deploy the 3D tracking technology and the 3D visualization of the surgical field in a manner that allows the surgeon to focus his attention (sight and hands) on the operatory field and on the patient rather than on separate screen. Similarly, using example embodiments described below, the surgeon is not required to interpolate movements that appear on an external display in one orientation on the fly to properly respond with movements in the actual working area.

### Computing Platform

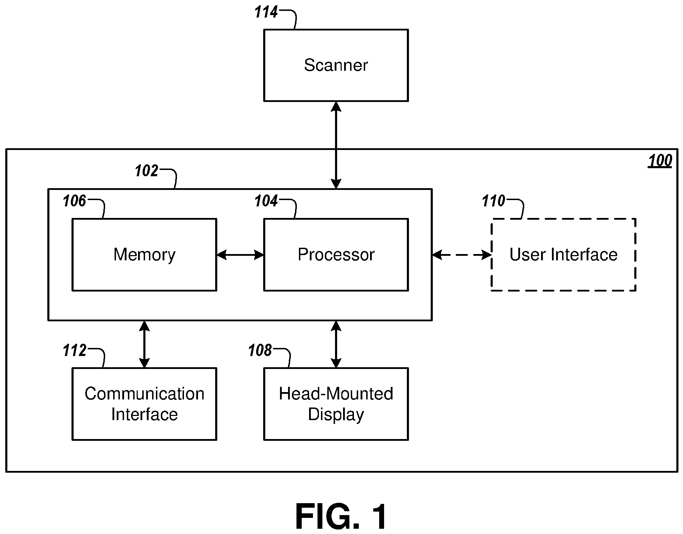

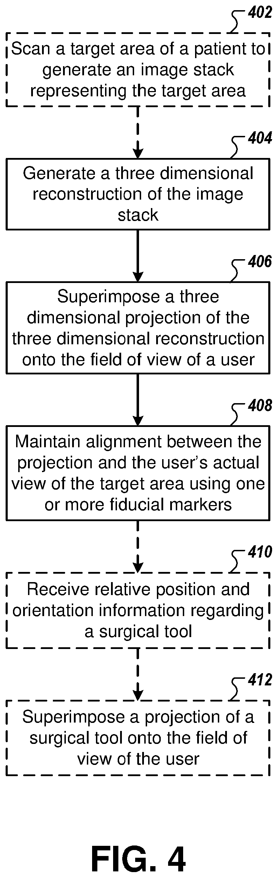

Figure 1 shows an example system that may perform the operations described herein for utilizing three dimensional augmented reality visualizations to assist surgery. In this regard, it is contemplated that computing device 100 may be configured to perform various operations in accordance with example embodiments of the present invention, such as in conjunction with the operations described in conjunction with Figures 4 through 8 below. It should be noted that the components, devices, and elements described herein may not be mandatory in every embodiment of the computing device 100, and some may be omitted in certain embodiments. Additionally, some embodiments may include further or different components, devices or elements beyond those shown and described herein.

As shown in Figure 1, the computing device 100 may include or otherwise be in communication with a processing system including, for example, processing circuitry 102 that is configurable to perform actions in accordance with example embodiments described herein. The processing circuitry 102 may be configured to perform data processing, application execution and/or other processing and management services according to an example embodiment of the present invention. In some embodiments, the computing device 100 or the processing circuitry 102 may be embodied as a chip or chip set. In other words, the computing device 100 or the processing circuitry 102 may comprise one or more physical packages (e.g., chips) including materials, components and/or wires on a structural assembly (e.g., a baseboard). The computing device 100 or the processing circuitry 102 may, in some cases, be configured to implement an embodiment of the present invention on a single chip or as a single "system on a chip." As such, in some cases, a chip or chipset may constitute means for performing one or more operations for providing the functionalities described herein.

In an example embodiment, the processing circuitry 102 may include a processor 104 and memory 106 that may be in communication with or otherwise control a head-mounted display 108 and, in some embodiments, a separate user interface 110. The processing circuitry 102 may further be in communication or otherwise control a scanner 114 (which may be external to the computing device 100 or, in some embodiments, may be included as a component of the computing device 100). In these capacities, the processing circuitry 102 may be embodied as a circuit chip (e.g., an integrated circuit chip) configured (e.g., with hardware or a combination of hardware and software) to perform or direct performance of operations described herein.

The processor 104 may be embodied in a number of different ways. For example, the processor 104 may be embodied as various processing means such as one or more of a microprocessor or other processing element, a coprocessor, a controller or various other computing or processing devices including integrated circuits such as, for example, an ASIC (application specific integrated circuit), an FPGA (field programmable gate array), or the like. In an example embodiment, the processor 104 may be configured to execute program code instructions stored in the memory 106 or otherwise accessible to the processor 104. As such, whether configured by hardware or by a combination of hardware and software, the processor 104 may represent an entity (e.g., physically embodied in circuitry) capable of performing operations according to embodiments of the present invention while configured accordingly. Thus, for example, when the processor 104 is embodied as an ASIC, FPGA or the like, the processor may include specifically configured hardware for conducting the operations described herein. Alternatively, as another example, when the processor 104 is embodied as an executor of software instructions, the instructions may specifically configure the processor 104 to perform the operations described herein.

The memory 106 may include one or more non-transitory memory devices such as, for example, volatile and/or non-volatile memory that may be either fixed or removable. The memory 106 may be configured to store information, data, applications, instructions or the like for enabling the computing device 100 to carry out various functions in accordance with example embodiments of the present invention. For example, the memory 106 could be configured to buffer input data for processing by the processor 104. Additionally or alternatively, the memory 106 could be configured to store instructions for execution by the processor 104. As yet another alternative, the memory 106 may include one of a plurality of databases that may store a variety of files, contents or data sets. Among the contents of the memory 106, applications may be stored for execution by the processor 104 in order to carry out the functionality associated with each respective application. In some cases, the memory 106 may be in communication with the processor 104 via a bus for passing information among components of the computing device 100.

The head-mounted display 108 includes one or more interface mechanisms for augmenting a user's view of the real-world environment. The head-mounted display 108 may, in this regard, comprise augmented reality glasses that augment a user's perceived view of the real-world environment. This effect can be achieved in a number of ways. For instance, in some embodiments, the augmented reality glasses may include a display (e.g., an LED or other similar display) in one lens (or both lenses) that produces a video feed that interleaves computer-generated sensory input with the view captured by a camera on the front of the lens (or lenses) of the augmented reality glasses. In such embodiments, the user cannot see directly through the lens (or lenses) providing this interleaved video feed, and instead only sees the interleaved video feed itself. In other embodiments, the user can see through the lenses of the augmented reality glasses, and a display is provided in a portion of one lens (or both) that illustrates computer-generated sensory input relating to the user's view. In yet other embodiments, the computer-generated sensory input may be projected onto a lens (or both lenses) by a projector located in the augmented reality glasses. In such embodiments, the lens (or lenses) may comprise a partially reflective material that beams the projected video into the eye(s) of the user, thus adding the computer-generated sensory input directly to the user's natural view through the lenses of the augmented reality glasses without disrupting the natural view.

The head-mounted display 108 may further include spatial awareness sensors (e.g., gyroscope, accelerometer, compass, or the like) that enable the computing system 100 to compute the real-time position and orientation of the head-mounted display 108.

In some embodiments, the head-mounted display 108 may itself incorporate the rest of the computing device 100, and thus the various elements of the computing device 100 may communicate via one or more buses. In other embodiments, the head-mounted display 108 may not be co-located with the rest of the computing device 100, in which case the head-mounted display may communicate with the computing device 100, by a wired connection, or by a wireless connection, which may utilize short-range communication mechanisms (e.g., infrared, Bluetooth^{™}, or the like), a local area network, or a wide area network (e.g., the Internet). In embodiments utilizing wireless communication, the head-mounted display 108 may include, for example, an antenna (or multiple antennas) and may support hardware and/or software for enabling this communication.

A separate user interface 110 (if needed) may be in communication with the processing circuitry 102 and may receive an indication of user input and/or provide an audible, visual, mechanical or other output to the user. As such, the user interface 110 may include, for example, a keyboard, a mouse, a joystick, a display, a touch screen, a microphone, a speaker, or any other input/output mechanisms. The computing device 100 need not always include a separate user interface 110. For example, in some embodiments the head-mounted display 108 may be configured both to provide output to the user and to receive user input (such as, for instance, by examination of eye motion or facial movements made by the user). Thus, a separate communication interface 110 may not be necessary. As such, the communication interface 110 is shown in dashed lines in Figure 1.

Communication interface 112 includes means such as a device or circuitry embodied in either hardware, or a combination of hardware and software that is configured to receive and/or transmit data between any device or module of the computing device 100 and any external device, such as by a wired connection, a local area network, or a wide area network (e.g., the Internet). In this regard, the head-mounted display 108 may include, for example, an antenna (or multiple antennas) and may support hardware and/or software for enabling communications with a wireless communication network and/or a communication modem or other hardware/software for supporting this communication.

The scanner 114 may be a tomograph or any other device that can use penetrating waves to capture data regarding an object. For instance, scanner 114 may comprise a computed tomography (CT) scanner, a positronic emission tomography (PET) scanner, a single-photon emission computed tomography (SPECT) scanner, or a magnetic resonance imaging (MRI) scanner. The scanner 114 is configured to produce a tomogram, which constitutes a two dimensional image illustrating a slice, or section, of the imaged object. In a typical scenario, however, the scanner 114 may produce a series of tomograms (referred to herein as an image stack) that, when analyzed in combination, can be used to generate a three dimensional mesh representative of the scanned object.

### Example Techniques for Utilizing Augmented Reality Visualization to Assist Surgery

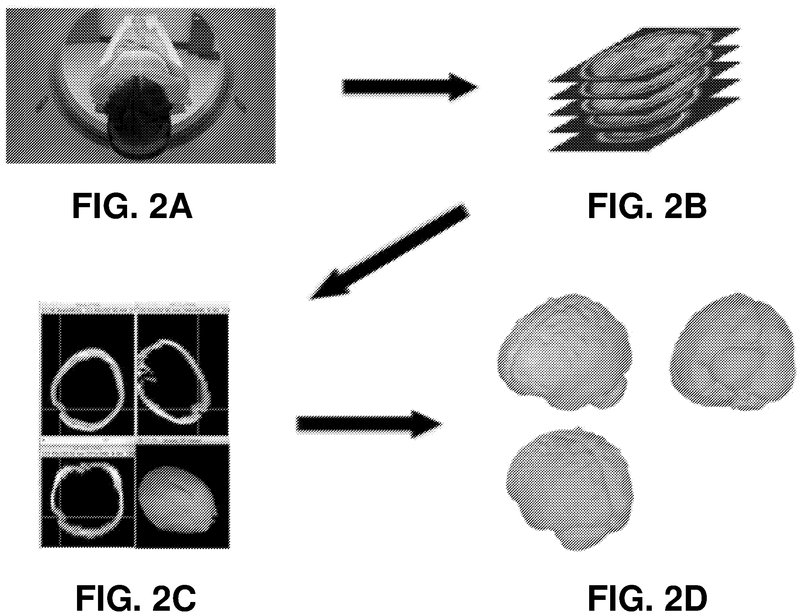

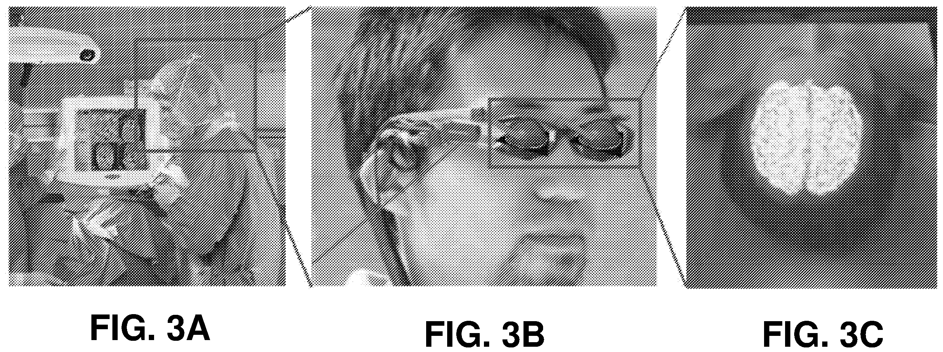

Having described above some example components that might be employed in the present invention, a description of a high-level procedure utilized by example embodiments will be provided in connection with a discussion of Figures 2A through 2D and 3A through 3C.

Turning first to Figures 2A through 2D, a series of images are shown illustrating an order of operations used to generate a 3D reconstruction of a target area (in these illustrations, the example used is a human brain). Starting first with Figure 2A, a patient must undergo a scan (using scanner 114 described above) to generate an image stack of one part of the patient's body. In this regard, Figure 2A illustrates a patient in a MRI scanner, although any other tomographic scanner (or alternative type of scanner) is contemplated, so long as it can produce an image stack (i.e., a series or sequence of images showing an object of interest) with mm resolution that can subsequently be converted into a 3D mesh. Figure 2B depicts a series of images forming an image stack that can subsequently be used to generate the 3D mesh.

Figure 2C illustrates the importation of the image stack into application software that can be used to generate a 3D reconstruction of the target area via image segmentation and mesh generation. In one example, a 3D reconstruction of a target area can be performed by loading the image stack into iLastik (a software application for biological image segmentation). The image stack can thus be segmented using the carving module of iLastik, which allow fast, accurate, semi-automated segmentation of the z stack, and to extract a mesh of the 3D object.

In turn, Figure 2D illustrates a visual representation of a 3D mesh generated in accordance with example embodiments described in greater detail below. Once this mesh is extracted, its size (in meters or centimeters) may need to be adjusted. Also, eventual compression on the z axis might need to be taken into account, if the voxel size of the 3D mesh is not isotropic (due, for instance to possible difference between the thickness of the optical slides and the xy resolution). This can be achieved using a Blender add-on (e.g., Neuromorph, a free collection of add-ons for Blender 3D modeling software, which allows importing 3D meshes and resizing them if the voxel size is known).

At this point, the 3D object of the target area can thus comprise a simple file in standard .obj format, which can then be loaded into augmented reality (AR) glasses (e.g., via a USB cable, SD card, or the like). The surgeon can then load the 3D model into augmented reality software within augmented reality glasses.

Turning next to Figures 3A through 3C, a sequence of images are illustrates that demonstrate the connection between various elements of the present invention utilized in example embodiments described herein. In Figure 3A, a surgeon is illustrated utilizing traditional assistive machinery for surgery. In this regard, Figure 3A demonstrates the use of a 2D representation of a target area (in this case, a brain) that requires the surgeon to alternately view the surgical site and then an external display illustrating the target area. In contrast, the surgeon can utilize augmented reality glasses, such as those shown in Figure 3B, to address this deficiency of traditional assistive machinery. In this regard, the augmented reality glasses of example embodiments of the present invention project the three dimensional model of the target area onto the field of view of the surgeon. Figure 3C illustrates an example augmented reality display enabled using augmented reality glasses.

### Operations Performed to Utilize Augmented Reality Visualization to Assist Surgery

Figures 4 through 8 illustrate flowcharts containing a series of operations performed by example embodiments described herein for utilizing augmented reality visualization to assist surgery. The operations shown in Figures 4 through 8 are performed in an example embodiment using a computing device 100 that may be embodied by or otherwise associated with processing circuitry 102 (which may include a processor 104 and a memory 106), a head-mounted display 108, and in some embodiments, a separate user interface 110 and scanner 114.

Turning first to Figure 4, a flow diagram illustrates example operations for utilizing three dimensional augmented reality visualizations to assist surgery.

In optional operation 402, the computing device 100 may include means, such as scanner 114 or the like, for scanning a target area to generate an image stack representing the target area. As described previously, the scanner 114 may be a tomograph or any other device that can use penetrating waves to capture image data regarding an object. For instance, scanner 114 may comprise a computed tomography (CT) scanner, a positronic emission tomography (PET) scanner, a single-photon emission computed tomography (SPECT) scanner, or a magnetic resonance imaging (MRI) scanner. The scanner 114 is configured to produce a tomogram, which constitutes a two dimensional image illustrating a slice, or section, of the imaged object. In a typical scenario, however, the scanner 114 may produces a series of tomograms (referred to herein as an image stack) that, when analyzed in combination, can be used to generate a three dimensional mesh representative of the scanned object.

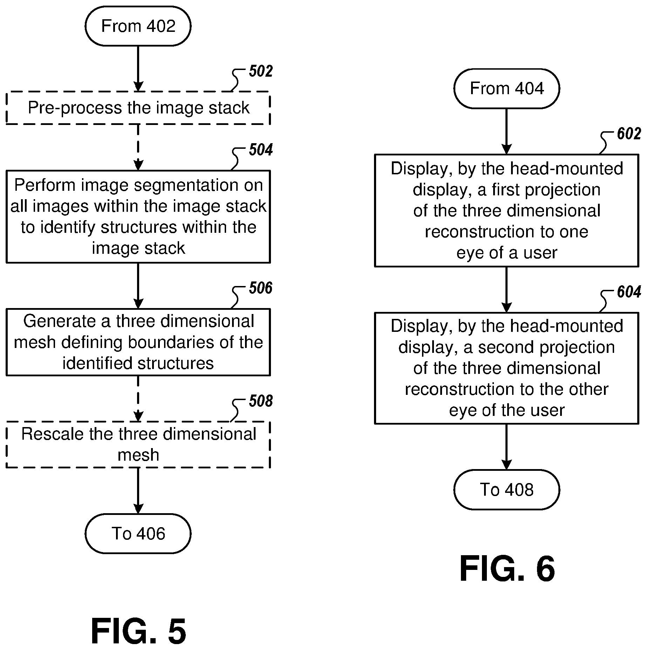

In operation 404, the computing device 100 includes means, such as processing circuitry 102 or the like, for generating a three dimensional reconstruction of an image stack representing a target area. The image stack may be received from a scanner 114 or from a local memory (e.g., memory 106) or from a remote memory via a communication interface (not shown in Figure 1). Generation of the 3D reconstruction is described in greater detail below in conjunction with Figure 5.

In operation 406, a head-mounted display 108 (or other similar wearable device), superimposes a 3D projection of the 3D reconstruction onto the field of view of the user (e.g., surgeon). The head-mounted display 108 may in some embodiments be an element of the computing device 100, although in other embodiments, the head-mounted display 108 may be a separate apparatus connected to the computing device 100 via wireless communication media, as described previously. Specific aspects of superimposing the 3D projection are described in greater detail below in connection with Figure 6.

In operation 408, the computing device 100 includes means, such as processing circuitry 102, head-mounted display 108, user interface 110, or the like, for maintaining alignment between the projection and the user's actual view of the target area using a plurality of fiducial markers associated with the target area. In this regard, these fiducial markers are deployed into the environment in advance of surgery and provide reference points that enable accurate relative positioning of the surgeon, surgical tools, and provide a better understanding of the location, orientation, and swelling of target areas. To this end, a subset of the fiducial markers may be pre-existing within the operating room environment while another subset of the fiducial markers may be inserted or otherwise disposed on different portions of the patient. In the latter respect, a subset of the plurality of fiducial markers may be located directly on the target area that is the subject of surgery (e.g., the brain), or on related tissue, such as on secondary target areas whose movement and swelling are intended to be tracked.

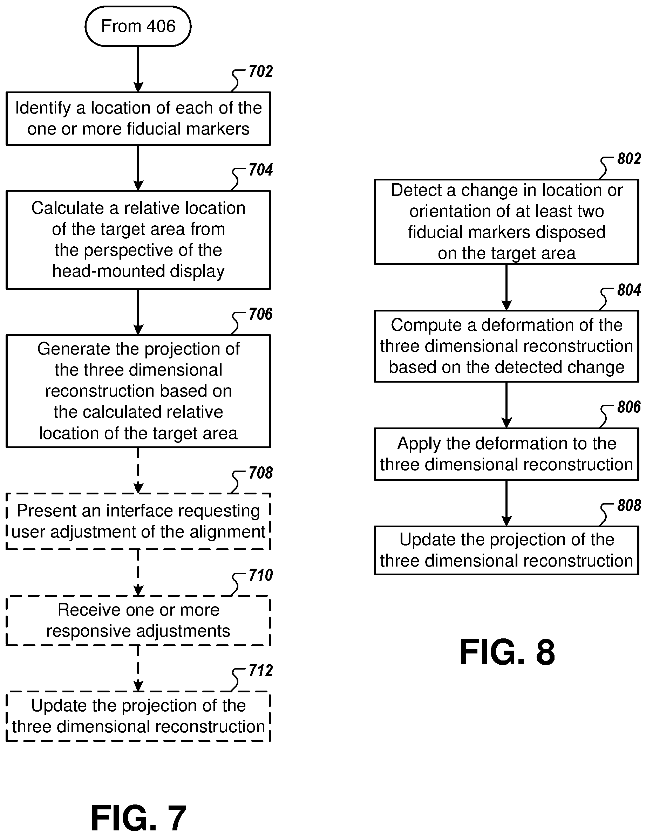

In addition to placing these fiducial markers in the operatory environment, the location of these markers must also be digitally placed into the 3D model during the preprocessing step so that the physical locations exactly match the corresponding positions in the virtual world. The camera in the augmented reality glasses can then recognize the fiducial markers in the real world, and match them with their digital representation. This will allow superimposing the real and the virtual world correctly. At least one of these fiducial markers should be placed directly on the organ of interest (i.e. brain). Because the organ can move and swell, this marker will be moving and follow the deformation of the organ, allowing the system to compute a deformation that can be applied to the 3D mesh loaded into the system. In this latter regard, specific operations for maintaining alignment of the 3D projection and the user's actual view of the target area are described in greater detail below in connection with Figures 7 and 8.

In optional operation 410, the computing device 100 includes means, such as processing circuitry 102, communication interface 110, or the like, for receiving relative position and orientation information regarding a surgical tool. To this end, assistive machinery (e.g., a neuronavigator or the like) utilizes surgical tools that are not directly visible during operations. Such assistive machinery generally utilizes its own tracking mechanisms to identify relative locations and orientations of the surgical tools and which can accordingly provide the spatial information of the position of the surgical tools inside that are not visible during a surgical procedure.

Example embodiments can therefore interface with the existing neuronavigation technologies that already provide feedbacks about the position of surgical tools to visualize their respective location. In this fashion, optional operation 410 enables the retrieval of this location and orientation from the assistive machinery via communication interface 110 or from a local memory (e.g., memory 106 of the processing circuitry 102).

Finally, in optional operation 412, the computing device 100 includes means, such as processing circuitry 102, head-mounted display 108, or the like, for superimposing a projection of the surgical tool onto the field of view of the user. Having received the location and orientation of surgical tools from the assistive machinery, the surgical tools can be projected onto the field of view of the surgeon in the same manner as described above in connection with operation 406 and below in connection with Figure 6.

Turning now to Figure 5, example operations are illustrated for generating a three dimensional reconstruction of a target area based on an image stack received from a scan of the target area, in accordance with some example embodiments of the present invention.

From optional operation 402 above (in embodiments in which operation 402 is performed) or in response to retrieval of an image stack from a memory store, the procedure may turn to operation 502, in which the computing device 100 includes means, such as processing circuitry 102 or the like, for pre-processing the image stack. In this regard, pre-processing the image stack may in some embodiments include aligning images of the image stack. In addition, pre-processing the image stack may in some embodiments include filtering images of the image stack to increase the SNR (signal-to-noise ratio) of the images.

Subsequently, in operation 504, the computing device 100 includes means, such as processing circuitry 102 or the like, for performing image segmentation on all images of the image stack to identify structures within the image stack. In this regard, image segmentation may utilize a seeded watershed algorithm. The algorithm can detect the edges of each image to be segmented based on that image's gradient, then afterwards each edge can be followed on the 3^{rd} dimension (z) by following the spatial direction of the edge on the sequence of images. The procedure may, in some embodiments, be iterated for the both a target area (e.g., an organ) and a lesion (previously recognized by the physician) that appears within the reconstructed organ, removal of which may be the primary purpose of the surgical procedure.

In operation 506, the computing device 100 includes means, such as processing circuitry 102 or the like, for generating a 3D mesh defining boundaries of the identified structures. This 3D mesh may comprise a collection of points and surfaces in a 3D space that approximate the shape of the segmented structure. In this regard, the 3D reconstruction discussed above in connection with Figure 4 may comprise this 3D mesh.

Scanner 114 typically provides information about pixel size and z thickness of an image stack. Thus, by reference to this information, the relative thickness of the images in the image stack and the XY resolution of the images of the image stack can be evaluated to determine if the generated 3D mesh includes isotropic voxels, in which case the 3D mesh can be utilized to present a 3D projection to the user. However, when the voxel size of the 3D mesh is not isotropic, a common result is that the Z dimension of the 3D mesh may be compressed or elongated.

In such circumstances, optional operation 508 may be performed, in which the computing device 100 includes means, such as processing circuitry 102 or the like, for rescaling the three dimensional mesh to render its voxels isotropic (correcting the compression or elongation caused by the asymmetry between the Z axis thickness and the XY-axis resolution of the images in the image stack.

Turning now to Figure 6, example operations are illustrated for superimposing a three dimensional augmented reality projection using augmented reality glasses, in accordance with some example embodiments of the present invention. It should be understood that while the operations of Figure 6 illustrate one example by which a 3D projection can be displayed, other embodiments may be contemplated, such as those discussed above.

In operation 602, the computing device 100 includes means, such as head-mounted display 108 or the like, for displaying a first projection of the three dimensional reconstruction to one eye of the user

In operation 604, the computing device 100 includes means, such as head-mounted display 108 or the like, for displaying a second projection of the three dimensional reconstruction to the other eye of the user. In this regard, it should be understood that the differences between the first projection and the second projection are designed to generate the three dimensional projection of the three dimensional reconstruction.

The 3D augmented reality projection may change over time with movement of the head-mounted display. In this regard, it should be understood that in order for the model to be superimposed correctly to the target area and to change perspective according to the relative position of the surgeon with respect to the target area, the augmented reality glasses utilize a front camera configured to recognize fiducial markers placed in the operating room that the computing device 100 can recognize as fixed points in the environment whose position relative to the patient is known. This enables correct alignment of the 3D model, as well as the tracking of the surgeon's head position. Similarly, tracking of the augmented reality glasses can be improved using sensors disposed on the augmented reality glasses (e.g., gyroscope(s), accelerometer(s), or the like) embedded in the augmented reality glasses.

Accordingly, turning now to Figure 7, example operations are illustrated for ensuring accurate alignment of a three dimensional projection based on relative locations and orientations of objects within a user's field of view, in accordance with some example embodiments of the present invention.

In operation 702, the computing device 100 includes means, such as processing circuitry 102 or the like, for identifying, by a camera associated with the head-mounted display, a location of each of the plurality of fiducial markers.

In operation 704, the computing device 100 includes means, such as processing circuitry 102 or the like, for calculating a relative location of the target area from the perspective of the head-mounted display.

In operation 706, the computing device 100 includes means, such as head-mounted display 108 or the like, for generating the projection of the three dimensional reconstruction based on the calculated relative location of the target area.

In optional operation 708, the computing device 100 includes means, such as head-mounted display 108, user interface 110, or the like, for presenting an interface requesting user adjustment of the alignment between the projection and the user's actual view of the target area.

In optional operation 710, the computing device 100 includes means, such as head-mounted display 108, user interface 110, or the like, for receiving one or more responsive adjustments. In this regard, the use of the fiducial markers should be as accurate as possible, in order to grant a perfect tracing of the position of the 3D model relative to the real world. Nevertheless, because the model will be superimposed on the eyes of the surgeon, the surgeon may be provided with the ability to finely adjust the position of the model by using a graphical interface, to correct millimetric adjusting errors (on the x, y, or z axes).

Thus, in optional operation 712, the computing device 100 includes means, such as processing circuitry 102, head-mounted display 108, or the like, for updating the projection of the three dimensional reconstruction in response to receiving the one or more responsive adjustments. These adjustments may be made u

In addition to the operations described above in connection with Figure 7, maintenance of the alignment of the projection can also take into account fiducial markers located in or near the target area can be examined to enable the model to follow the natural displacements and deformation of the brain itself. The 3D projection, which is based on the 3D mesh, can thus follow the deformation and modify its volume according to the actual movement of the brain. In some example embodiments, this spatial tracking can be achieved utilizing an existing SDK (software development kit), provided by Vuforia, wikitude, or Metaio. Those SDKs are able to track the spatial position and orientation of the camera in the augmented reality glasses relative to the known fiducial markers.

Turning now to Figure 8, example operations are illustrated for maintaining alignment of a three dimensional projection based on changes in objects within the field of view, in accordance with some example embodiments of the present invention.

In operation 802, the computing device 100 includes means, such as processing circuitry 102 or the like, for detecting a change in location or orientation of at least two fiducial markers disposed on the target area, wherein movement of the at least two fiducial markers disposed on the target area indicates a change in location or shape of the target area.

In operation 804, the computing device 100 includes means, such as processing circuitry 102 or the like, for computing a deformation of the three dimensional reconstruction based on the detected change. Subsequently, in operation 806, the computing device 100 includes means, such as processing circuitry 102 or the like, for applying the deformation to the three dimensional reconstruction. Finally, in operation 808, the computing device 100 includes means, such as processing circuitry 102 or the like, for updating the projection of the three dimensional reconstruction in response to applying the deformation to the three dimensional reconstruction.

In addition, it should be understood that this alignment can be improved through the use of input coming from the neuronavigation system, which provides an additional spatial reference to the target area with respect to the surgical tools utilized during surgery.

Accordingly, the operations illustrated in Figures 4 through 8 provide a procedure that utilizes augmented reality visualization to assist surgery. In particular, utilizing augmented reality 3D glasses, example embodiments deploy 3D tracking and 3D visualization of the surgical field in a manner that allows the surgeon to focus attention (sight and hands) on the operatory field and on the patient rather than on separate screen. Similarly, using example embodiments, the surgeon is not required to interpolate movements that appear on an external display in one orientation on the fly to properly respond with movements in the actual working area. Accordingly, example embodiments described herein provide significant benefits for surgical applications having little or no direct visibility and surgical applications utilizing assistive machinery.

The above-described flowcharts illustrate operations performed by an apparatus (which include the hardware elements of computing device 100 of Figure 1), in accordance with some example embodiments of the present invention. It will be understood that each block of the flowchart, and combinations of blocks in the flowchart, may be implemented by various means, such as hardware, firmware, processor, circuitry and/or other device associated with execution of software including one or more computer program instructions. For example, one or more of the procedures described above may be embodied by program code instructions. In this regard, the program code instructions which embody the procedures described above may be stored by a memory 106 of the computing device 100 employing an embodiment of the present invention and executed by a processor 104 of the computing device 100. As will be appreciated, loading these program code instructions onto a computing device 100 produces a specially programmed apparatus configured to implement the functions specified in the respective flowchart blocks. The program code instructions may also be stored in a non-transitory computer-readable storage medium that may direct a computer or other programmable apparatus to function in the prescribed manner, such that storing the program code instructions in the computer-readable storage memory produce an article of manufacture which can be accessed by an computing device 100 to implement the functions specified in the flowchart blocks. Accordingly, the operations illustrated in the flowchart define algorithms for configuring a computer or processing circuitry 102 (e.g., a processor) to perform example embodiments described above. When a general purpose computer stores the algorithms illustrated above, the general purpose computer is transformed into a particular machine configured to perform the corresponding functions.

Blocks of the flowchart support combinations of means for performing the specified functions and combinations of operations for performing the specified functions. It will be understood that one or more blocks of the flowchart, and combinations of blocks in the flowchart, can be implemented by special purpose hardware-based computer systems which perform the specified functions, or combinations of special purpose hardware and computer instructions.

Many modifications and other embodiments of the invention set forth herein will come to mind to one skilled in the art to which the invention pertains having the benefit of the teachings presented in the foregoing descriptions and the associated drawings. Although the foregoing descriptions and the associated drawings describe certain example combinations of elements and/or functions, it should be appreciated that different combinations of elements and/or functions may be provided by alternative embodiments without departing from the scope of the appended claims. Although specific terms are employed herein, they are used in a generic and descriptive sense only and not for purposes of limitation.

## Claims

1. A system for utilizing augmented reality visualization in surgery, the system comprising:
a plurality of fiducial markers comprising:
a first subset of the plurality of fiducial markers configured to be placed in an operating room as fixed points whose position relative to a patient is known, and
a second subset of the plurality of fiducial markers configured to be placed on a target area of a patient;
an apparatus (100) configured to generate (404) a three dimensional reconstruction of an image stack representing the target area of the patient by
performing (504) image segmentation on all images of the image stack to identify structures within the image stack, and
generating (506) a three dimensional mesh defining boundaries of the identified structures, wherein
the three dimensional reconstruction comprises the three dimensional mesh, wherein, when the fiducial markers have been placed, the apparatus is additionally configured to digitally place the corresponding locations of a digital representation of the plurality of fiducial markers into the three dimensional reconstruction of the image stack;
a head-mounted display (108) configured to superimpose (406) a three dimensional projection of the three dimensional reconstruction onto a field of view of a user; and
a camera in the head-mounted display configured to identify (702) a location of each of the plurality of fiducial markers, and to match the plurality of fiducial markers with their digital representation, so that the apparatus is configured to maintain (408) alignment between the projection and the user's actual view of the target area using the plurality of fiducial markers,
wherein the camera is configured to detect (802) a change in the location of the second subset of the plurality of fiducial markers indicating a change in location or
shape of the target area, so that the apparatus is configured to compute (804) a deformation of the three dimensional reconstruction, and the head-mounted display is configured to update (808) the projection of the three dimensional reconstruction.

2. The system of claim 1, wherein the apparatus is configured to maintain alignment between the projection and the user's actual view of the target area by
calculating a relative location of the target area from the perspective of the head-mounted display, and
generating the projection of the three dimensional reconstruction based on the calculated relative location of the target area.

3. The system of claim 1, further comprising:
a scanner that scans the body part to generate the image stack, wherein the scanner is one of:
a computed tomography (CT) scanner that scans the target area;
an magnetic resonance imaging (MRI) scanner that scans the target area;
a positronic emission tomography (PET) scanner that scans the target area; or
a single-photon emission computed tomography (SPECT) scanner that scans the target area.

4. The system of claim 1, wherein the generation by the apparatus of the three dimensional reconstruction of the image stack representing the target area includes:
pre-processing the image stack.

5. The system of claim 4, wherein pre-processing the image stack by the apparatus includes at least one of:
aligning images of the image stack; or
filtering images of the image stack.

6. The system of claim 1, wherein, in an instance in which a voxel size of the three dimensional mesh is not isotropic, the apparatus rescales the three dimensional mesh.

7. The system of claim 1, wherein head-mounted display superimposes the three dimensional projection of the three dimensional reconstruction onto the field of view of the user by:
displaying a first projection of the three dimensional reconstruction to one eye of the user; and
displaying a second projection of the three dimensional reconstruction to the other eye of the user,
wherein differences between the first projection and the second projection are designed to generate the three dimensional projection of the three dimensional reconstruction.

8. The system of claim 1, wherein the apparatus maintains alignment between the projection and the user's actual view of the target area further by:
presenting a user interface requesting user adjustment of the alignment between the projection and the user's actual view of the target area.

## Patentansprüche

1. System zur Verwendung von Visualisierung erweiterter Realität in Chirurgie, das System umfassend:
eine Vielzahl von Passermarken, umfassend:
eine erste Untergruppe der Vielzahl von Referenzmarkern, die konfiguriert sind, um in einem Operationssaal als feste Punkte platziert zu werden, deren Position in Bezug auf einen Patienten bekannt ist, und
eine zweite Untergruppe der Vielzahl von Passermarken, die konfiguriert sind, um auf einem Zielbereich eines Patienten platziert zu werden;
eine Vorrichtung (100), die konfiguriert ist, um eine dreidimensionale Rekonstruktion eines Bildstapels zu erzeugen (404), der den Zielbereich des Patienten darstellt, durch
Durchführen (504) einer Bildsegmentierung an allen Bildern des Bildstapels, um Strukturen innerhalb des Bildstapels zu identifizieren, und
Erzeugen (506) eines dreidimensionalen Netzes, das Grenzen der identifizierten Strukturen definiert, wobei die dreidimensionale Rekonstruktion das dreidimensionale Netz umfasst,
wobei, wenn die Passermarken platziert worden sind, die Vorrichtung zusätzlich konfiguriert ist, um die entsprechenden Stellen einer digitalen Darstellung der Vielzahl von Passermarken in der dreidimensionalen Rekonstruktion des Bildstapels digital zu platzieren;
eine kopfgetragene Anzeige (108), die konfiguriert ist, um eine dreidimensionale Projektion der dreidimensionalen Rekonstruktion auf ein Sichtfeld eines Benutzers zu überlagern (406); und
eine Kamera in der kopfgetragenen Anzeige, die konfiguriert ist, um eine Stelle von jeder der Vielzahl von Passermarken zu identifizieren (702) und die Vielzahl von Passermarken mit ihrer digitalen Darstellung abzugleichen, sodass die Vorrichtung konfiguriert ist, um eine Ausrichtung zwischen der Projektion und der tatsächlichen Sicht des Benutzers auf den Zielbereich unter Verwendung der Vielzahl von Passermarken aufrechtzuerhalten (408),
wobei die Kamera konfiguriert ist, um eine Änderung der Stelle der zweiten Untergruppe der Vielzahl von Referenzmarkierungen zu erkennen (802), die eine Änderung der Stelle oder der Form des Zielbereichs angibt, sodass die Vorrichtung konfiguriert ist, um eine Deformation der dreidimensionalen Rekonstruktion zu berechnen (804), und die am Kopf getragene Anzeige konfiguriert ist, um die Projektion der dreidimensionalen Rekonstruktion zu aktualisieren (808).

2. System nach Anspruch 1, wobei die Vorrichtung konfiguriert ist, um eine Ausrichtung zwischen der Projektion und der tatsächlichen Sicht des Benutzers auf den Zielbereich aufrechtzuerhalten, indem sie eine relative Stelle des Zielbereichs aus der Perspektive der kopfgetragenen Anzeige berechnet, und
Erzeugen der Projektion der dreidimensionalen Rekonstruktion basierend auf der berechneten relativen Stelle des Zielbereichs.

3. System nach Anspruch 1, ferner umfassend:
einen Scanner, der den Körperteil abtastet, um den Bildstapel zu erzeugen, wobei der Scanner einer von Folgenden ist:
ein Scanner für Computertomographie (CT), der den Zielbereich abtastet;
ein Scanner für Magnetresonanztomograph (MRI), der den Zielbereich abtastet;
ein Scanner für Positronen-Emissions-Tomographie (PET), der den Zielbereich abtastet; oder
ein Scanner für Einzelphotonen-Emissions-Computertomographen (SPECT), der den Zielbereich abtastet.

4. System nach Anspruch 1, wobei die Erzeugung, durch die Vorrichtung, der dreidimensionalen Rekonstruktion des Bildstapels, der den Zielbereich darstellt, Folgendes beinhaltet:
Vorverarbeiten des Bildstapels.

5. System nach Anspruch 4, wobei ein Vorverarbeiten des Bildstapels durch die Vorrichtung mindestens eines von Folgenden beinhaltet:
Ausrichten von Bildern des Bildstapels; oder
Filtern von Bildern des Bildstapels.

6. System nach Anspruch 1, wobei in einem Fall, in dem eine Voxelgröße des dreidimensionalen Netzes nicht isotrop ist, die Vorrichtung das dreidimensionale Netz neu skaliert.

7. System nach Anspruch 1, wobei die kopfgetragene Anzeige die dreidimensionale Projektion der dreidimensionalen Rekonstruktion auf das Sichtfeld des Benutzers überlagert, durch:
Anzeigen einer ersten Projektion der dreidimensionalen Rekonstruktion für ein Auge des Benutzers; und
Anzeigen einer zweiten Projektion der dreidimensionalen Rekonstruktion für das andere Auge des Benutzers,
wobei die Unterschiede zwischen der ersten Projektion und der zweiten Projektion konstruiert sind, um die dreidimensionale Projektion der dreidimensionalen Rekonstruktion zu erzeugen.

8. System nach Anspruch 1, wobei die Vorrichtung eine Ausrichtung zwischen der Projektion und der tatsächlichen Sicht des Benutzers auf den Zielbereich aufrechterhält, ferner durch:
Darstellen einer Benutzeroberfläche, die den Benutzer zur Anpassung der Ausrichtung zwischen der Projektion und der tatsächlichen Sicht des Benutzers auf den Zielbereich auffordert.

## Revendications

1. Système permettant d'utiliser la visualisation par réalité augmentée en chirurgie, le système comprenant :
une pluralité de marqueurs de repère comprenant :
un premier sous-ensemble de la pluralité de marqueurs de repère configurés pour être placés dans une salle d'opération en tant que points fixes dont la position par rapport à un patient est connue, et
un deuxième sous-ensemble de la pluralité de marqueurs de repère configuré pour être placé sur une zone cible d'un patient ;
un appareil (100) configuré pour générer (404) une reconstruction tridimensionnelle d'une pile d'images représentant la zone cible du patient par
réalisation (504) d'une segmentation d'image sur toutes les images de la pile d'images pour identifier les structures au sein de la pile d'images, et
génération (506) d'un maillage tridimensionnel définissant les limites des structures identifiées, la reconstruction tridimensionnelle comprenant le maillage tridimensionnel,
dans lequel, lorsque les marqueurs de repère ont été placés, l'appareil est en outre configuré pour placer de façon numérique les emplacements correspondants d'une représentation numérique de la pluralité de marqueurs de repère dans la reconstruction tridimensionnelle de la pile d'images ;
un visiocasque (108) configuré pour superposer (406) une projection tridimensionnelle de la reconstruction tridimensionnelle sur un champ de vision d'un utilisateur ; et
une caméra dans le visiocasque configurée pour identifier (702) un emplacement de chacun de la pluralité de marqueurs de repère, et pour faire correspondre la pluralité de marqueurs de repère avec leur représentation numérique, de sorte que l'appareil est configuré pour maintenir (408) l'alignement entre la projection et la vue réelle de l'utilisateur de la zone cible à l'aide de la pluralité de marqueurs de repère,
dans lequel la caméra est configurée pour détecter (802) un changement de l'emplacement du deuxième sous-ensemble de la pluralité de marqueurs de repère indiquant un changement d'emplacement ou de forme de la zone cible, de sorte que l'appareil est configuré pour calculer (804) une déformation de la reconstruction tridimensionnelle, et le visiocasque est configuré pour mettre à jour (808) la projection de la reconstruction tridimensionnelle.

2. Système selon la revendication 1, dans lequel l'appareil est configuré pour maintenir l'alignement entre la projection et la vue réelle de l'utilisateur de la zone cible par
calcul d'un emplacement relatif de la zone cible du point de vue du visiocasque, et
génération de la projection de la reconstruction tridimensionnelle en fonction de l'emplacement relatif calculé de la zone cible.

3. Système selon la revendication 1, comprenant en outre :
un scanner qui balaye la partie du corps pour générer la pile d'images, le scanner étant l'un parmi :
un scanner de tomodensitométrie (TDM) qui balaye la zone cible ;
un scanner d'imagerie par résonance magnétique (IRM) qui scanne la zone cible ;
un scanner de tomographie par émission de positons (TEP) qui balaye la zone cible ; ou
un scanner de tomographie par émission monophotonique (SPECT) qui balaye la zone cible.

4. Système selon la revendication 1, dans lequel la génération par l'appareil de la reconstruction tridimensionnelle de la pile d'images représentant la zone cible comprend :
le prétraitement de la pile d'images.

5. Système selon la revendication 4, dans lequel le prétraitement de la pile d'images par l'appareil comprend au moins l'un parmi :
l'alignement des images de la pile d'images ; ou
le filtrage des images de la pile d'images.

6. Système selon la revendication 1, dans lequel, dans un cas dans lequel une taille de voxel du maillage tridimensionnel n'est pas isotrope, l'appareil redimensionne le maillage tridimensionnel.

7. Système selon la revendication 1, dans lequel le visiocasque superpose la projection tridimensionnelle de la reconstruction tridimensionnelle sur le champ de vision de l'utilisateur par :
affichage d'une première projection de la reconstruction tridimensionnelle à un oeil de l'utilisateur ; et
affichage d'une deuxième projection de la reconstruction tridimensionnelle à l'autre oeil de l'utilisateur,
dans lequel les différences entre la première projection et la deuxième projection sont conçues pour générer la projection tridimensionnelle de la reconstruction tridimensionnelle.

8. Système selon la revendication 1, dans lequel l'appareil maintient en outre l'alignement entre la projection et la vue réelle de l'utilisateur de la zone cible par :
présentation d'une interface utilisateur demandant à l'utilisateur d'ajuster l'alignement entre la projection et la vue réelle de l'utilisateur de la zone cible.
